# EUROPEAN PATENT APPLICATION

(11) **EP 4 163 926 A1**
(43) Date of publication of application: **12.04.2023**
(21) Application number: 21200948.4
(22) Date of filing: 05.10.2021
(51) Int. Cl.: G16H 20/70

(54) **SYSTEM AND METHOD FOR MONITORING THE REACTION OF A USER AND ADJUSTING OUTPUT CONTENT ACCORDINGLY**

(71) Applicant: Koa Health B.V., 08018 Barcelona (ES)
(72) Inventor: Garcia i Tormo, Albert, 08013 Barcelona (ES); Hemmings, Nicola, Bristol, BS4 3AS (GB); Buda, Teodora Sandra, 08019 Barcelona (ES); Garriga Calleja, Roger, 08470 Barcelona (ES); Lucchesi, Federico, 00174 Rome (IT); Maffei, Giovanni, 08006 Barcelona (ES)
(74) Representative: Herrmann, Uwe

(57) **Abstract**

The present disclosure relates to a system for monitoring a reaction of a user and adjusting output content accordingly; the system comprising an output unit, configured to present content to a user; a monitoring unit configured to monitor a parameter of the user during a time period in that a first content is presented to the user via the output unit to obtain monitoring data; a synchronization unit, configured to synchronize the monitoring data obtained by the monitoring unit during the period in that the first content is presented by the output unit with the first content presented by the output unit to thereby link in time the monitoring data and the first content; an analysis unit, configured to analyze the monitoring data obtained by the monitoring unit and link the data to the first content presented to determine the user's reaction to the first content presented; and a control unit configured to control the output unit to present a second content to the user, wherein the second content is selected based on the determined reaction of the user to the first content. Further, the disclosure relates to a method for monitoring a reaction of a user and adjusting output content accordingly that preferably uses a system according to the present disclosure.

## Description

The present disclosure relates to a system and method for monitoring the reaction of a user to a given content and adjusting output content accordingly, preferably in real-time.

As the recent COVID-19 pandemic has doubled the rates of common mental health disorders such as depression and anxiety, there is a huge and growing unmet need to remedy undesired symptoms of mental health conditions in the population. It is estimated that around 1 in 5 (21%) adults experienced some form of depression in early 2021. This is an increase compared to since November 2020 (19%) and more than double that observed before theCOVID-19 pandemic (10%).

This increase of mental health conditions has put a strain on mental health care professionals such as therapists and psychologists whose number have remained constant. In addition to that, contact restrictions due to the pandemic have often exacerbated mental health disorders and also posed a hurdle to treatment, as patients could not easily meet mental health care professionals in person.

In addition to that, conventionally, standardised questionnaires, such as the WHO-5 well-being index, are the basis for the assessment of the mental state of a user. However, despite being the standard, the input gathered by such questionnaires is subjective and prone to biases and even misuse.

It is the object of the present invention to alleviate or completely abolish the draw-backs associated with the prior art. In particular, it is an object of the present inven-tion to ensure that all people receive adequate assistance with their mental health conditions without putting an undue strain on mental health care professionals and to ensure that the mental state of each user is accurately determined.

A system or method according to the present disclosure can ensure that the mental health state of an individual user is accurately determined and the individual user receives tailored mental health care recommendations and resources (e.g. a customized content can be displayed during desensitization treatment for anxiety disorders), that meet the user wherever he / she is in his / her clinical trajectory and do so as early as possible in that journey.

An aspect of the invention relates to a system for monitoring a reaction of a user, for example a reaction of the user to a given content, and adjusting output content accordingly; the system comprising:
- An output unit, configured to present content to a user;
- a monitoring unit configured to monitor a parameter of the user during a time period in that a first content is presented to the user via the output unit to obtain monitoring data;
- a synchronization unit, configured to synchronize the monitoring data obtained by the monitoring unit during the period in that the first content is presented by the output unit with the first content presented by the output unit to thereby link in time the monitoring data and the first content;
- an analysis unit, configured to analyze the monitoring data obtained by the monitoring unit and link the data to the first content presented to determine the user's reaction to the first content presented; and
- a control unit configured to control the output unit to present a second content to the user, wherein the second content is selected based on the determined reaction of the user to the first content.

As an example, the system may be used to expose a user to a series of images that are expected to elicit a certain reaction in the user, for example fear of the user when presented with an image of a spider. Depending on the detected reaction of the user to a given image, e.g. a detected moderate reaction (moderately increased heart rate) to a realistic image of a spider, the next image to be presented to the user will be an image expected to elicit a stronger response of the user, e.g. an image of a realistic spider sitting on a human hand expected to elicit a strongly increased heart rate in the user.

The present disclosure, however, is not limited to a case in that a defined series of content to be presented is used. Instead the control unit can also be configured to select the next content based on a reaction of the user to a previous content. In this case, the series of content presented is not defined in advance but is decided instantaneously. For example, the first content is selected from a first pool of contents and the second content is selected from a second pool of contents based on a detected reaction of the user to the first content, e.g. through the monitoring of physiological data or based on input the user actively provides. In other words, in this case, there is no pre-defined order of contents to be presented but the series rather evolves gradually based on the reactions of the user to content previously presented.

The monitoring unit may be configured to monitor one or more of the following parameters: a physiological parameter of the user, such as heart rate, respiration rate, pupil dilation, body temperature, skin conductivity; a behavioral parameter, such as an activity profile, sleep pattern, a reaction time, gaze direction, data regarding social interactions and a parameter data reflecting a conscious state of the user, such as data stemming from questionnaires or data input by the user.

The monitoring unit can comprise one or more sensors configured to measure the parameters to be monitored. The data acquired by the monitoring unit when monitoring the one or more parameters are referred to as monitoring data.

For example, a system according to the present invention may be realized in a smartphone. Smartphones include multiple sensors which can also be used for user monitoring, for example whilst the user is using the phone. The sensors can provide automatic and unobtrusive measurements of physiological parameters of the user. In particular front camera of a user's smartphone can be used to monitor different physiological parameters, as this camera provides a close-up of the patient's face whilst they are using the phone. These parameters include, but are not limited to the instantaneous heart rate (from a photoplethysmogram signal, camera-based measurement) and / or the instantaneous respiration rate (movement around the chest area, camera-based measurement). The main advantage of using e.g. a camera for monitoring physiological parameters is that they can be completely unobtrusive and automatic, thereby allowing to monitor users without the measurement influencing them (unconditioned measurements) and without requiring them to explicitly provide input.

A monitoring unit to monitoring a parameter of the user automatically and unobtrusively without the user being required to actively provide input, offers the possibility of monitoring users who cannot easily fill up text-based questionnaires, such as children or people with difficulties to read, and to extend the text-based questionnaires with non-textual questions. For instance, images or videos can be presented to the user and the user's reaction to them can be monitored, wherein data reflecting the user's reaction is synchronized or linked in time with the presented content.

In addition to that, the monitoring unit can be configured to monitor the parameter of the user for any desired time period, e.g. continuously 24 hours a day, only during the time period in that a first content is displayed, for a given number of hours each day etc.

According to an embodiment, the analysis unit is configured to receive data reflecting a conscious state of the user, such as data stemming from questionnaires or data input by the user, and data reflecting a subconscious state of the user, such as physiological data, and to compare the data reflecting the conscious state of the user with the data reflecting the subconscious state of the user to determine the user's reaction to the first content presented.

For example, the user may consciously report not fear but the physiological data may indicate signs of fear, such as an increased heart rate. Taking both data reflecting the conscious state of the user, such as data stemming from questionnaires or data input by the user, and data reflecting a subconscious state of the user, such as physiological data or behavioral data, into account allows for a more accurate detection of the user's reaction.

According to an embodiment, the analysis unit is configured to detect changes in the parameter monitored by the monitoring unit relative to a previous measurement and to determine the user's reaction to the first content presented based on the detected changes.

Alternatively or additionally, the analysis unit is configured to detect the absolute value of the parameter monitored by the monitoring unit and to determine the user's reaction to the first content presented based on the detected absolute value.

According to an embodiment, the control unit is configured to select the second content during a time period in that the first content is displayed and / or is configured to control the output unit to present the second content immediately after the first content.

In other words, the control unit may be configured to select the second content in real time, e.g. during the time period in that the first content is displayed and / or is configured to control the output unit in real time to present the second content immediately after the first content.

Alternatively, the control unit is configured to select the second content during a time period in that the first content is displayed and / or is configured to control the output unit to present the second content in the future, e.g. the next time the user interacts with the system according to the present disclosure. In this case, the content presented is not immediately adapted based on the detected reaction of the user, but instead, data reflecting the detected reaction of the user is stored and the selected second content is presented at a desired time in the future. For example, the second time a user interacts with the system according to the invention, a different series of first content and second content is presented than the first time the user interacted with the system.

According to an embodiment of the system, the control unit is configured to select the second content to elicit a desired reaction of the user. For example, the control unit can select a second content expected to elicit a stronger reaction of the user (e.g. a strong increase in heart rate) or a second content expected to elicit a milder reaction of the user (e.g. a mild increase in heart rate). In other words, the second content may be selected to induce a desired reaction in the user or to put the user in a desired mental state, e.g. to induce a desired level of fear or wellbeing.

According to an embodiment, the control unit is configured to select a second content expected to elicit a stronger physiological reaction of the user than the first content, if the determined physiological reaction of the user to the first content lies within a defined tolerance range, and / or wherein the control unit is configured to select a second content expected to elicit a milder physiological reaction of the user than the first content, if the determined physiological reaction of the user to the first content lies outside of, in particular exceeds, a defined tolerance range, and / or wherein the control unit is configured to select a second content expected to calm the user, in particular a guided relaxation program, if the determined physiological reaction of the user to the first content lies outside of, in particular exceeds, a defined tolerance range.

According to an embodiment, the monitoring unit, the output unit and the synchronization unit are present in one single device, such as a smartphone, comprising a synchronization device, such as an internal clock, and the synchronization unit is configured to use the signal of the synchronization device to synchronize the monitoring data obtained by the monitoring unit with the first and / or second content presented by the output unit. The synchronization can involve linking in time the monitoring data with the content presented, e.g. by providing corresponding monitoring data and data regarding the presented content with a common time stamp.

The system according to the present disclosure may further comprise a memory in that a determined series of contents to be consecutively presented to the user via the output unit is stored, wherein the control unit is configured to control the output unit to consecutively present the contents of the series to the user and if the reaction of the user determined by the analysis unit to a presented content of the determined series exceeds a defined tolerance range, to interrupt and / or modify the consecutive presentation of contents.

The tolerance range preferably is defined in terms of the monitoring data and may for example comprise a maximum value for the heart rate or respiration rate or a minimum value of the sleep time in case of behavioral data being monitored.

In a system according to the present disclosure, the analysis unit can be further configured to automatically determine the mental health state of the user based on the determined user's reaction to the first content presented. If, for example, the user shows a strong reaction to the first content that is expected to elicit only a mild reaction the analysis unit can determine that the user is in a general state of agitation or stress in that even relatively mild stimuli elicit a strong reaction. Conversely, if the user is in a relaxed and happy state, a content that is expected to elicit a strong reaction may elicit only a mild reaction.

Another aspect of the disclosure relates to a method for monitoring the reaction of a user and adjusting output content accordingly; the method preferably employing a system according to the present disclosure, the method comprising the steps:
- presenting to a user a first content via an output unit;
- monitoring a parameter of the user during a time period in that the first content is presented to the user via the output unit to obtain monitoring data;
- synchronizing the data regarding the parameter obtained by the monitoring unit during a period in that the first content is presented by the output unit with the first content presented by the output unit via a synchronization unit to thereby link in time the monitoring data and the first content;
- analyzing the monitoring data obtained by the monitoring unit and linking these data to the first content presented to determine the user's reaction to the first content presented by means of an analysis unit; and
- controlling via a control unit the output unit to present a second content to the user, wherein the second content is selected by the control unit based on the determined reaction of the user to the first content.

According to an embodiment, the monitoring step comprises monitoring one or more of the following parameters: a physiological parameter of the user, such as heart rate, respiration rate, pupil dilation, body temperature, skin conductivity; a behavioral parameter, such as data regarding an activity profile, sleep pattern, a reaction time, gaze direction, data regarding social interactions and a parameter reflecting a conscious state of the user, such as data stemming from questionnaires or data input by the user. Other data, such as data stemming from electronic health records may also be acquired via the monitoring unit.

The method may further comprise the steps: receiving via the analysis unit data reflecting a conscious state of the user, such as data stemming from questionnaires or data input by the user, and data reflecting a subconscious state of the user, such as physiological data and comparing the data reflecting the conscious state of the user with the data reflecting the subconscious state of the user to determine the user's reaction to the first content presented.

According to an embodiment, the method may comprise the step of: by means of the analysis unit detecting changes in the parameter monitored by the monitoring unit relative to a previous measurement and determining the user's reaction to the first content presented based on the detected changes.

Alternatively or additionally, the analysis unit may be used for detecting an absolute value of the parameter monitored by the monitoring unit and determining the user's reaction to the first content presented based on the detected absolute value.

Preferably, the second content is selected during a time period in that the first content is displayed and / or the output unit is controlled to present the second content immediately after the first content.

In other words, the second content may be selected in real time, e.g. during the time period in that the first content is displayed and / or the output unit may be controlled in real time to present the second content immediately after the first content. Alternatively, the second content may be selected during a time period in that the first content is displayed and the output unit is controlled to present the second content in the future, e.g. the next time the user interacts with the system according to the present disclosure. In this case, the content presented is not immediately adapted based on the detected reaction of the user, but instead, data reflecting the detected reaction of the user or data reflecting the selected second content is stored and the selected second content is presented at a desired time in the future. For example, the second time a user interacts with his / her smartphone, according to a method of the present disclosure a different series of first content and second content is presented than the first time the user interacted with his / her smartphone.

In a method according to the present disclosure, the second content is preferably selected to elicit a desired reaction in the user. For example, the control unit can select a second content expected to elicit a stronger reaction of the user (e.g. a strong increase in heart rate) or a second content expected to elicit a milder reaction of the user (e.g. a mild increase in heart rate). In other words, the second content may be selected to induce a desired reaction in the user or to put the user in a desired mental state, e.g. to induce a desired level of fear or wellbeing.

For example, according to an embodiment of the method, a second content expected to elicit a stronger physiological reaction of the user than the first content is selected, if the determined physiological reaction of the user to the first content lies within a defined tolerance range, and / or wherein a second content expected to elicit a milder physiological reaction of the user than the first content is selected, if the determined physiological reaction of the user to the first content lies outside of, in particular exceeds, a defined tolerance range, and / or wherein a second content expected to calm the user is selected, in particular a guided relaxation program, if the determined physiological reaction of the user to the first content lies outside of, in particular exceeds, a defined tolerance range.

According to an embodiment of the method, the synchronizing step is performed using a signal of a synchronization device, such as an internal clock, of a single device comprising the monitoring unit, the output unit and the synchronization unit to synchronize the monitoring data obtained by the monitoring unit with the first and / or second content presented by the output unit. For example, the single device is a smartphone.

According to an embodiment, the method comprises a step of storing in a memory a determined series of contents to be consecutively presented to the user via the output unit, and a step of controlling the output unit to consecutively present the contents of the series to the user and, if the reaction of the user determined by the analysis unit to a presented content of the determined series exceeds a defined tolerance range, to interrupt and / or modify the consecutive presentation of contents.

As will be apparent to the user skilled in the art, any effects, features or elements described in the context of the system according to the present disclosure equally apply to the method according to the present disclosure even if not explicitly recited herein to reduce redundancy. Similarly, any effects, features or elements recited herein may be taken in isolation or combined in any desired way, as is apparent to the person skilled in the art.

Further effects, features or elements of the present disclosure will become apparent from the following description of embodiments of the invention taking reference to the figures.
Fig. 1 shows an exemplary embodiment of a system and method according to the present disclosure;
Fig. 2 shows an example of an iterative analysis by the analysis unit of the monitoring data obtained by the monitoring unit;
Fig. 3 shows an exemplary embodiment of a system and method according to the present disclosure including exemplary physiological data;
Fig. 4 shows an exemplary sequence of contents to be presented to the user based on the determined reaction of the user.

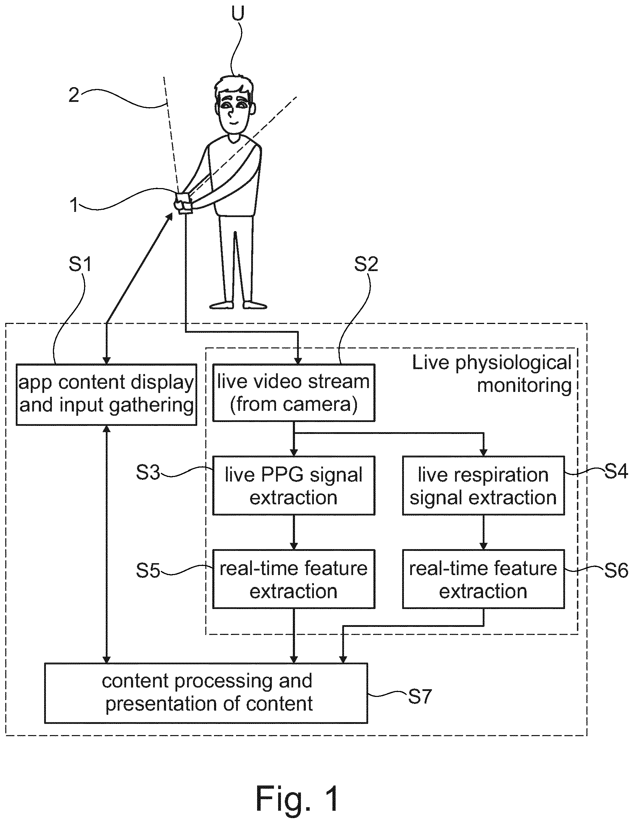

As shown in Fig. 1, a user U interacts with his smartphone 1 holding the smartphone such that the face and chest of the user are present in an acquisition range 2 of the smart phone in that the front camera of the smart can be used to monitor parameters, for example physiological parameters, of the user. In this example, a system according to the present invention is realized as the smartphone 1 of the user. The smart phone 1 of the user in other words is used to perform a method according to the present disclosure.

In step S1, the output unit, in this case the screen of the smartphone, presents the user U with a content, e.g. via an application. At the same time, the app can prompt the user to rate his current mental state or state of wellbeing or to provide any other input reflecting the conscious state of the user.

While the user U is interacting with the smartphone 1, in step S2 a live video stream is acquired using the front camera of the smartphone 1 to monitor the user. Preferably, a color video stream is acquired. The acquisition of the video stream in S2 does not require any active input of the user and might not even be noticed by the user.

In step S3, from the video stream a signal indicating the heart rate of the patient, e.g. a photoplethysmogram (PPG), is acquired. In general, different vital signs can be monitored with regular cameras, including pulse and respiration, as well as activity, sleep and other aspects related to the user's health. A photoplethysmogram (PPG) is the optical measurement of blood volume changes in the microvascular bed of tissue. In other words, PPG is the measurement of changes of color in the skin caused by the pulsatile flow of blood flowing through the body. With each heart beat, the heart pumps a pulse of blood through the arteries; the blood pulse travels through them to the capillaries and, from there, it returns to the heart via the veins. Since the skin has many capillaries (i.e. it is highly perfused), it is feasible to optically measure the pulsatility of the blood flow; this is, whenever a blood pulse reaches the capillaries, the local increase in the blood flow causes a local increase of light absorption which, in turn, causes a minute color change in the skin. Even though this color change is imperceptible to the naked eye, it can be captured with a camera (PPG signal).
The PPG signal consists of a large constant (DC) component, which corresponds to the main skin absorption, and a pulsatile (AC) low-amplitude component, which corresponds to the variation in blood volume. Typically, the amplitude of the pulsatile component is in the range of 1%, compared to the constant component.

Generally, the amplitude of the pulsatile component is very low, even below the resolution in an 8-bit scale and well below the camera noise level. In order to reject the noise and achieve enough resolution, usually the signal is not measured from just one pixel but averaged over a large number of pixels (Region Of Interest, ROI). As the PPG signal is strongest at the areas which are highest perfused, the face and the palms of the hands and the feet are usually the best areas to measure the PPG signal. The raw PPG signal shows variations in light intensity: a burst of blood increases the absorption which results in a decrease of light intensity. The peaks of the raw PPG signal correspond to the moments of minimum blood flow. Color cameras capture three different wavelengths: red, green and blue. The light absorption is largest around the green wavelength, which results in a PPG signal of larger amplitude in the green channel than in the blue and the red ones. Thus, preferably, the green channel of the camera is analyzed to measure the user's heart rate. In a healthy subject, all blood pulses pumped by the heart reach all limbs and, in particular, the face and the hand. Consequently, measuring the frequency of the PPG signal at, e.g. the hand, is a way of measuring the heart rate. Furthermore, since the Pulse Transit Time (PTT) does not substantially affect the cycle to cycle measurement, it is feasible to measure the Instantaneous Heart Rate (this is, the length of each individual heart cycle, iHR) to evaluate parameters such as the Heart Rate Variability (HRV). Insights about other parameters such as blood pressure can be obtained as well from the PPG signal.

In step S4, from the video stream a signal indicating the respiration rate of the patient is acquired. The respiration rate can be extracted from the video stream by monitoring the chest movement of the user.

The signal extraction in steps S3 and S4 occurs live during the acquisition of the video stream and concurrently with the display of a first content on the smartphone. Thus, the data obtained in step S2 and analyzed subsequently in steps S3-S6 is synchronized with the first content that is displayed to the user.

In steps S5 and S6 features are extracted from the PPG signal extracted in step S3 and the respiratory signal extracted in step S4. The purpose of this is to extract a defined parameter corresponding to a defined numerical that can be used for numerical processing from the video stream acquired in step S2. For example, in step S5, a heart rate in beats per minute (bpm) is extracted from the pulsatile changes in tissue color encoded in the PPG signal. In step S6, for example, a respiration rate in breaths per minute is extracted from the chest movements detected in S4.

The feature extraction in steps S5 and S6 in this example occurs in real time, i.e. at the same time at that the first content is displayed on the smart phone 1.

Of course, it would also be possible, that the feature extraction and analysis occurs not in real time but with a time delay relative to the acquisition of the monitoring data. For example, the monitoring data could be stored in a memory for later processing. The monitoring data could then be processed even during a time period in that the user does not interact with his smart phone and the content to be presented at the next time the user interacts with the smartphone could be determined.

In step S7, the features extracted in steps S5 and S6 are linked in time with the content presented on the smartphone 1, so that the physiological reaction of the user in terms of heart rate and respiration rate can be linked to the first content.

In step S7 then a second content to be presented to the user is selected based on the detected physiological reaction of the user to the first content. The second content is then presented to the user U on the display of the smartphone 1.

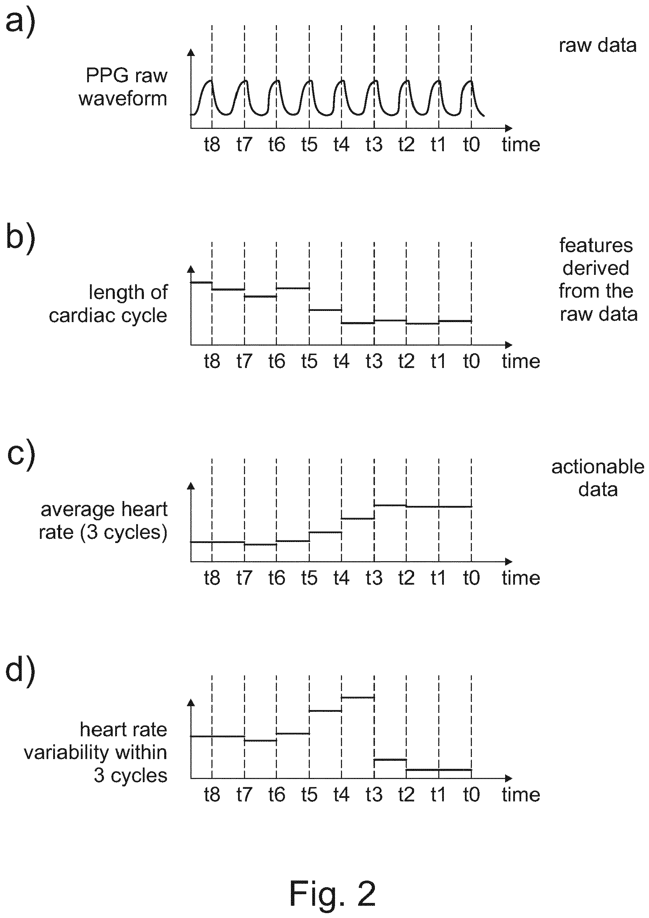

Fig. 2 shows an example of an iterative analysis performed on PPG data acquired by the front camera of the smartphone 1 of the user U to extract features useful for subsequent processing (also referred to as actionable data).

In Fig. 2a) raw data is shown, in this example the wave form indicating color changes of the user's skin acquired in step S3. In step S5, this raw data is processed to extract the length of the cardiac cycle from the PPG signal as shown in panel 2b) and to obtain actionable data such as the average heart rate of the user in bpm averaged over three cycles as shown in panel 2c) or the average heart rate variability in ms averaged over three cycles as shown in panel 2d). For example, the average heart rate shown in Fig. 2c) and the average heart rate variability shown in Fig. 2d can then be used to determine the user's reaction to the first content. For example, panel 2c) shows an increase in average heart rate from t8 to t0 that can indicate that the user is experiencing the first content presented as agitating or arousing.

A live measurement of the heart rate can be obtained in the following way: The face of the user needs to be located in the acquisition range of the camera. This can be easily achieved with a face detector. Likewise, the face detector can also identify elements in the face, such as the forehead and the cheeks. These three elements (forehead and both cheeks) define the Region of Interest (Rol) and they need to be identified in all frames of the video stream acquired by the front camera. The raw PPG signal can be extracted from the live video stream by averaging all the pixels within the Rol per frame. For an improved signal-to-noise ratio, the green, red and blue channels may be independently analysed and afterwards combined. The result of each frame can be concatenated thereby creating a time-domain signal (raw PPG signal).

Directly relying the raw PPG signal to determine the user's reaction is not advisable because the information that it conveys is implicit within a waveform (and thus not actionable) that captures multiple physiological parameters at the same time. The raw PPG signal is thus be split into multiple signals, each of which conveying explicit information of only one physiological feature, such as the heart rate or the heart rate variability (actionable data). A feasible way of obtaining actionable data from the raw PPG signal is to determine the length of each cardiac cycle; this is, to locate the peaks in the raw PPG signal, which correspond to the moments of minimum blood flow, and then evaluate the time distance between peaks (length of the cardiac cycle). This feature can be further split into an Average Heart Rate (e.g. the invert of the average length of the last three cardiac cycles, aHR) and the HRV (the length difference between the last two cardiac cycles). These features convey explicit information of only one physiological parameter and therefore they are actionable and can be used to determine the user's reaction to the content displayed.

Using a device such as the smartphone 1, the process of feature extraction from raw data can be executed with a very low delay, so that e.g. the cardiac information is updated and made available for processing within a few milliseconds after each heart beat. This allows that, as soon as there is a change in the patient's heart beat, it will be immediately detected by the smartphone 1.

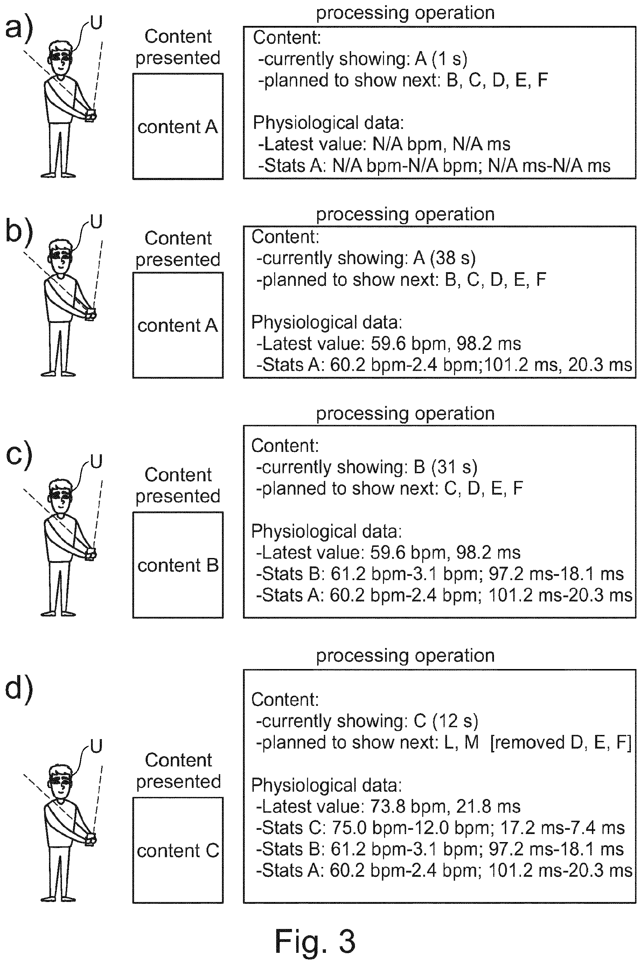

Fig. 3 shows an exemplary embodiment of a system and method according to the present disclosure that monitors the user U via the front camera of his smartphone 1 and obtains PPG data from that the average heart rate over there cycles and the heart rate variability over three cycles are extracted. The reaction of the user U to a content presented to the user is detected based on changes in the extracted heart rate and hart rate variability that occur right after the patient is being presented with a content. In this example, there is a defined series of contents to be consecutively presented to the user, contents A-F.

As shown in panel 3a), during a first stage at the beginning of the interaction of the user his smartphone 1 the user u is viewing a content A presented on the smartphone 1. The content A has been presented to the user for 1s. A live video stream of the user is being acquired via the front camera of the smartphone 1. As the interaction of the user u with the smartphone 1 has only started, there are no data yet available for the average heart rate in bpm and the average heart rate variability in ms.

As shown in panel 3b), the content A has been presented to the user U for 38s. Data indicating the average heart rate in bpm and the average heart rate variability in ms have been extracted from the live video stream. For example, the most current instantaneous heart rate of the user is 59.6 bpm and the latest heart rate variability is 98.2 ms. In addition to that, statistical analysis has been performed to obtain the average heart rate, in this example 60.2 bpm and the standard deviation of this value, in this example 2.4 bpm, and the average heart rate variability, in this example 101.2 ms and the standard deviation of this value, in this example 20.3.

The acquired physiological data are associated with the content A that has been presented during the acquisition of the data by pooling. Statistical analysis in then performed on the data in each pool.

Pooling refers to creating a set of pools of data, one for each different content being presented in the application. For example, there is a pool of data for content A, a pool of data for content B and so on. The data acquired in panel 3b) is pooled into a pool associated with content A.

Each new physiological datapoint (i.e. each new value of average heart rate (aHR) and heart rate variability (HRV)), is stored in the corresponding pool according to the content presented.

Whenever a new value is added to a pool, two statistical parameters for each of aHR and HRV are evaluated for that pool: the average value and the standard deviation. The statistics are significant when at least e.g. five data points have been acquired for that pool.

To compare data, the following criterion can be used: given a datapoint a and a pool with average b and standard deviation c, a behaves similarly to that pool if a ∈ [b-c, b+c).

Based on this criterion, a criterion for determining the user's reaction to a presented content, for example, a potential increase in anxiety, can be defined: when, e.g. the aHR in the current pool is larger than that in the previous pool and the HRV in the current pool is smaller than that in the previous pool, this indicates that the presentation of the current content induced an increase in heart rate and a decrease in HRV relative to the previous content and thus elicited an increase in anxiety in the user.

The feature extraction and feature analysis may be performed by a machine learning-based system, such as a trained model or neural network, for example a convolutional neural network.

To establish the link between the change in the physiological features and the presented content, it is advantageous to identify the moment in time where these physiological changes occurred and link them to the content that was presented to the user at that moment. Since both the presentation of the content and the acquisition of the physiological data occur on the same device, for example, a synchronization device such as the internal clock can be used for synchronising the different data sources. Each datapoint can be accompanied by metadata, which can include at least a timestamp in the format of e.g. time since epoch in seconds; in this way, when comparing datapoints from different inputs (e.g. heart rate and change of presented content), it is only necessary to compare the timestamps to determine their chronological sequence.

In the example shown in Fig. 3b) the physiological data acquired when the user is being presented with content A is feeling relaxed. The aHR is low (around 60 bpm in average, with less than 5 bpm variation) and it exhibits Respiratory Sinus Arrhythmia (RSA), a respiration-induced modulation of the instantaneous heart frequency that is common when individuals are relaxed. Because of RSA, the HRV is large, in the range of 100ms.

After having displayed content A to the user for a defined time period, as shown in Fig. 3c) content B is displayed to the user u on his smartphone1. When switching to content B, the data pool for content A is closed and the pool for content B is opened, so any new data acquired will be grouped into the pool for content B. After gathering some data, e.g. at least three values of aHR, statistical analysis is performed on the data in the pool for content B. When viewing content B, the average heart rate is 61.2 bpm with a standard deviation of 3.1 bpm and the average heart rate variability is 97.2 ms with a standard deviation of 18.1 ms.

The statistical values derived from the data in the pool corresponding to contents A and B are compared and in this example are found to be similar or not significantly different. This is, the average value during B falls into the interval defined by A +/- the standard deviation (61.2 ∈ [57.8, 62.6) ). Also, all instantaneous values are found to be similar.

As shown in Fig. 3d), when the user is presented with content C, the values are no longer similar. It is not strictly necessary to acquire many heart cycles to derive statistics, because in this case the instantaneous values already indicate a different physiological response. Of course, it is possible to derive statistical values of the data points in the pool for content C as well. In this example, as shown in Fig. 3c) the average heart rage of the user viewing content C quickly increases beyond 70 bpm, reaching an instantaneous value of 73.8 bpm and an average value of 75.0 bpm, which is well above the maximum (average plus standard deviation) during the previous contents (62,6 bpm and 64,3 bpm respectively).

Likewise, the HRV also shows a significant change in response to content C, since the average value of the HRV when the user views content C, 17ms, is well below the average value minus standard deviation when the user views contents A and B, 80,9ms and 79,1ms, respectively.

Because of this change in both the aHR and HRV (both values deviating from the values known to be associated with a relaxed state, such as when viewing contents A and B), the reaction of the user to content C can be determined to be an increase in anxiety.

The control unit of the smartphone 1 thus adapts the series of contents to be consecutively presented to the user, for example by removing the initially planned contents D, E and F that are expected to be increasingly provocative to the user and adding the new contents L and M expected to e.g. calm the user. The series of content to be presented is thus adapted from A-B-C-D-E-F to A-B-C-L-M based on the detected reaction of the user (increase of anxiety) to content C.

In this example, the criterion to determine an anxiety increase is that both the aHR and the HRV must exceed a certain threshold. Different criteria may be used, which may involve using different physiological data and/or quantifying the measurement into actionable data (see next embodiment). The criteria may be defined as desired to detect and identify different reactions of the user.

Also, instead of providing a binary output (increase in fear: yes or no?), the criterion may provide a multi-level value (i.e. a number) indicating the intensity of detected reaction (e.g. rating of the detected increase in fear on a scale from 1-5).

For example, a possible multi-level quantification from only the aHR is to first determine a reference level and then provide as quantified output the difference of the current value with respect to the reference level; the higher the value, the higher the anxiety. The reference level can be acquired e.g. during the first 30 s, whilst the user is presented with some relaxing content, even though the reference value does not need to be acquired at every time the user interacts with his smartphone 1.

It is also possible to determine the normal or baseline values for an individual user by analyzing the physiological data over time, probably across multiple days. In this way, an individual starting point or baseline can be defined for an individual user. This baseline does not need to be necessarily based on numbers (e.g. the average heart rate when the patient is feeling relaxed), but they can also be based on other features. For instance, the shape of the PPG waveform. Monitoring multiple features at the same time introduces redundancy, which is often advisable in order to reduce the errors in conclusions drawn from the acquired data. Such personalized baseline recordings can also be used to determine which features are most relevant for each particular user, since not all features may equally indicative of a given reaction in individual users. For example, for a first user the increase in heart rate is more closely linked to an increase in anxiety and for a second user the increase in respiration rate is more closely linked to an increase in anxiety.

Any desired parameter of a user may be monitored to obtain data reflecting the user's reaction to a content displayed. Still referring to image processing, the amount of head movement or the pupil size are just two parameters. Furthermore, other data may be incorporated as well (synchronised with the content presented), such as accelerometer data, missed taps on the phone or tap intensity.

Another parameter that may provide valuable insights is the reaction time. The reaction time is defined as the elapsed time between a first and a second event. The first event can be the change in the content displayed to the user and the second event may be a change in a physiological feature; or the first event may be a change in a physiological feature and the second event a user input; or the first event may be a change in the content displayed in the application and the second event a user input.

The user's reaction may also be detectable from the user's voice. Indeed, since vocalization is entirely integrated within both the central and autonomic nervous system, there is a link between the voice output and the associated psychological and physiological state of the user. The voice data can be captured using a microphone, processed within milliseconds and then used as disclosed above.

Similarly to video-based signals, to analyse the voice or audio data (i.e. an audio signal), first the relevant features from the audio that can be linked to the target outcome have to be extracted; then e.g. an arousal level of the user can be derived by analysing these values and, if applicable, the content may be customised.

For example, stress can be detected by analyzing the voice of the patient. Some features that may be extracted are (non-exhaustive): respiration rate, articulation rate, word duration, vowel duration, respiration time between words or sentences, voice onset time, hitter, shimmer, signal to noise ratio, harmonic to noise ratio, mean F0 SD, F0 peaks or F0 floor values. Based on changes detected in the voice-based extracted features, the level of stress can be quantified, either in a binary way, or multi- level.

In addition to that, data from wearables or any other sources separate from the smartphone 1 may be used, for example a chest band or an additional PPG sensor in e.g. the hand may be used, and the data is made available in real time and synchronised with the smartphone.

Generally, based on the acquired data, mismatches between the physiological (and thus spontaneous) reaction of a user and the conscious reaction of the user (detected e.g. based on input provided by the patient) may be detected. For instance, in a situation where the user claims to feel nervous, he may be presented with some relaxing content and afterwards the patient claims to feel relaxed; however the physiological measurements indicate a state of higher anxiety (compared to what is normal for that patient, i.e. baseline data).

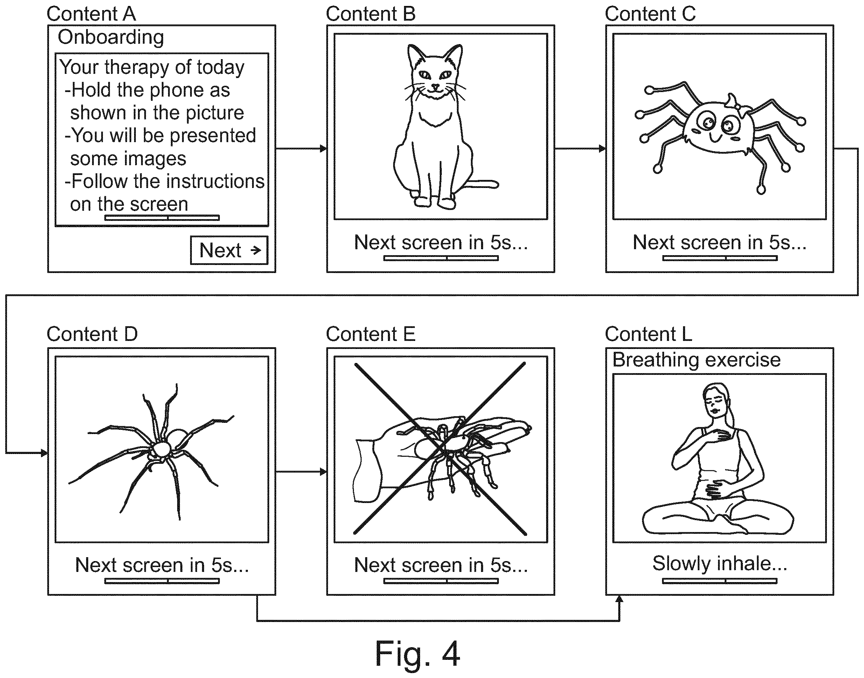

Fig. 4 shows an exemplary series of contents to be presented to a user suffering from fear of spiders based on the determined reaction of the user.

First, content A is presented to the user U on his smartphone 1. Content A has emotionally neutral content relating to instructions for the interaction with the smart phone. When viewing content A, the user is determined to be in a relaxed state and shows no significant physiological reaction.

Second, content B is presented to the user U on his smartphone 1. Content B is a picture of a cat and thus is expected to be emotionally neutral or pleasant to the user suffering from fear of spiders. When viewing content B, the user is determined to also be in a relaxed state and shows no significant physiological reaction.

Third, content C is presented to the user U on his smartphone 1. Content C is a cartoon picture of a spider and thus is expected to elicit only a very mild reaction in the user suffering from fear of spiders. When viewing content C, the user is determined to be in a state of very mild anxiety and shows only a very mild significant physiological reaction, e.g. a small increase in heart rate. The reaction of the user is still within a defined tolerance range, so the next content in the series is displayed next.

Thus, content D is presented to the user U on his smartphone 1. Content D is a realistic picture of a spider and thus is expected to elicit a moderate reaction in the user suffering from fear of spiders. When viewing content D, the user is determined to be in a state of strong anxiety and shows a strong significant physiological reaction, e.g. a large increase in heart rate. The reaction of the user to content D exceeds the defined tolerance range. As the reaction of the user to content D exceeds the defined tolerance range, not the next content in the defined series is presented to the user, but a different content, in this case content L is selected to be displayed next. The reason for that is that the content in the series is arranged to be increasingly provocative the user suffering from fear of spiders. However, if a certain level of exposure has been achieved and thus a certain reaction of the user outside of the tolerance range has been achieved, then it is not desirably to induce more fear in the user and the content to be displayed is adapted accordingly.

Following on content D thus content L is presented to the user, in this case a program guiding the user through a breathing exercise to calm the user. Content E that originally had been planned to be presented after content D is not presented, because this image of a spider sitting on a hand is expected to be even more provocative to the user and the user's reaction to content D already exceeded the tolerance range.

## Claims

1. System for monitoring a reaction of a user and adjusting output content accordingly; the system comprising:
- An output unit, configured to present content to a user;
- a monitoring unit configured to monitor a parameter of the user during a time period in that a first content is presented to the user via the output unit to obtain monitoring data;
- a synchronization unit, configured to synchronize the monitoring data obtained by the monitoring unit during the period in that the first content is presented by the output unit with the first content presented by the output unit to thereby link in time the monitoring data and the first content;
- an analysis unit, configured to analyze the monitoring data obtained by the monitoring unit and link the data to the first content presented to determine the user's reaction to the first content presented; and
- a control unit configured to control the output unit to present a second content to the user, wherein the second content is selected based on the determined reaction of the user to the first content.

2. System according to claim 1, wherein the monitoring unit is configured to monitor one or more of the following parameters: a physiological parameter of the user, such as heart rate, respiration rate, pupil dilation, body temperature, skin conductivity; a behavioral parameter, such as an activity profile, sleep pattern, a reaction time, gaze direction, data regarding social interactions and a parameter data reflecting a conscious state of the user, such as data stemming from questionnaires or data input by the user.

3. System according to claim 1 or 2, wherein the analysis unit is configured to receive data reflecting a conscious state of the user, such as data stemming from questionnaires or data input by the user, and data reflecting a subconscious state of the user, such as physiological data, and to compare the data reflecting the conscious state of the user with the data reflecting the subconscious state of the user to determine the user's reaction to the first content presented.

4. System according to one of the preceding claims, wherein the analysis unit is configured to detect changes in the parameter monitored by the monitoring unit relative to a previous measurement and to determine the user's reaction to the first content presented based on the detected changes.

5. System according to one of the preceding claims, wherein the control unit is configured to select the second content during a time period in that the first content is displayed and / or is configured to control the output unit to present the second content immediately after the first content.

6. System according to one of the preceding claims, wherein the control unit is configured to select the second content to elicit a desired reaction of the user.

7. System according to claim 6, wherein the control unit is configured to select a second content expected to elicit a stronger physiological reaction of the user than the first content, if the determined physiological reaction of the user to the first content lies within a defined tolerance range, and / or wherein the control unit is configured to select a second content expected to elicit a milder physiological reaction of the user than the first content, if the determined physiological reaction of the user to the first content lies outside of, in particular exceeds, a defined tolerance range, and / or wherein the control unit is configured to select a second content expected to calm the user, in particular a guided relaxation program, if the determined physiological reaction of the user to the first content lies outside of, in particular exceeds, a defined tolerance range.

8. System according to one of the preceding claims, wherein the monitoring unit, the output unit and the synchronization unit are present in one single device comprising a synchronization device, such as an internal clock, and the synchronization unit is configured to use the signal of the synchronization device to synchronize the monitoring data obtained by the monitoring unit with the first and / or second content presented by the output unit.

9. System according to one of the preceding claims, further comprising a memory in that at least one determined series of contents to be consecutively presented to the user via the output unit is stored, wherein the control unit is configured to control the output unit to consecutively present the contents of the series to the user and if the reaction of the user determined by the analysis unit to a presented content of the determined series exceeds a defined tolerance range, to interrupt and / or modify the consecutive presentation of contents.

10. System according to one of the preceding claims, wherein the analysis unit is further configured to automatically determine the mental health state of the user based on the determined user's reaction to the first content presented.

11. Method for monitoring the reaction of a user and adjusting output content accordingly; the method preferably employing a system according to one of claims 1-10 , the method comprising the steps:
- presenting to a user a first content via an output unit;
- monitoring a parameter of the user during a time period in that the first content is presented to the user via the output unit to obtain monitoring data;
- synchronizing the data regarding the parameter obtained by the monitoring unit during a period in that the first content is presented by the output unit with the first content presented by the output unit via a synchronization unit to thereby link in time the monitoring data and the first content;
- analyzing the monitoring data obtained by the monitoring unit and linking these data to the first content presented to determine the user's reaction to the first content presented by means of an analysis unit; and
- controlling via a control unit the output unit to present a second content to the user, wherein the second content is selected by the control unit based on the determined reaction of the user to the first content.

12. Method according to claim 11, wherein the monitoring step comprises monitoring one or more of the following parameters: a physiological parameter of the user, such as heart rate, respiration rate, pupil dilation, body temperature, skin conductivity; a behavioral parameter, such as data regarding an activity profile, sleep pattern, a reaction time, gaze direction, data regarding social interactions and a parameter reflecting a conscious state of the user, such as data stemming from questionnaires or data input by the user.

13. Method according to claim 12 or 13, further comprising the steps: receiving via the analysis unit data reflecting a conscious state of the user, such as data stemming from questionnaires or data input by the user, and data reflecting a subconscious state of the user, such as physiological data and comparing the data reflecting the conscious state of the user with the data reflecting the subconscious state of the user to determine the user's reaction to the first content presented.

14. System according to one claims 11-13, comprising the step of: by means of the analysis unit detecting changes in the parameter monitored by the monitoring unit relative to a previous measurement and determining the user's reaction to the first content presented based on the detected changes.

15. Method according to one of claims 11-14, wherein the second content is selected during a time period in that the first content is displayed and / or the output unit is controlled to present the second content immediately after the first content.

16. Method according to one of claims 11-14, wherein the second content is selected to elicit a desired reaction in the user.

17. Method according to claim 16, wherein a second content expected to elicit a stronger physiological reaction of the user than the first content is selected, if the determined physiological reaction of the user to the first content lies within a defined tolerance range, and / or wherein a second content expected to elicit a milder physiological reaction of the user than the first content is selected, if the determined physiological reaction of the user to the first content lies outside of, in particular exceeds, a defined tolerance range, and / or wherein a second content expected to calm the user is selected, in particular a guided relaxation program, if the determined physiological reaction of the user to the first content lies outside of, in particular exceeds, a defined tolerance range.

18. Method according to one of the preceding claims 11-17, wherein the synchronizing step is performed using a signal of a synchronization device, such as an internal clock, of a single device comprising the monitoring unit, the output unit and the synchronization unit to synchronize the monitoring data obtained by the monitoring unit with the first and / or second content presented by the output unit.

19. Method according to one of the preceding claims 11-18, further comprising the step of storing in a memory at least one determined series of contents to be consecutively presented to the user via the output unit, and
controlling the output unit to consecutively present the contents of the series to the user and, if the reaction of the user determined by the analysis unit to a presented content of the determined series exceeds a defined tolerance range, to interrupt and / or modify the consecutive presentation of contents.
